# EUROPEAN PATENT APPLICATION

(11) **EP 4 588 442 A1**
(43) Date of publication of application: **23.07.2025**
(21) Application number: 24152891.8
(22) Date of filing: 19.01.2024
(51) Int. Cl.: A61B 6/03, A61B 5/00, A61B 5/055, A61B 5/11, A61B 6/04, A61B 6/00, G06T 7/00, G16H 30/40, G06T 7/20, A61B 6/10

(54) **PATIENT MONITORING DURING A SCAN**

(71) Applicant: Koninklijke Philips N.V., 5656 AG Eindhoven (NL)
(72) Inventor: KRUEGER, Sascha, Eindhoven (NL); SENEGAS, Julien Thomas, Eindhoven (NL)
(74) Representative: Philips Intellectual Property & Standards

(57) **Abstract**

An imaging system is provided for capturing images of a patient during a medical scan using a medical scanner having a scanning system and a patient support. A camera is fixed relative to the scanning system and captures images of the patient support. Within the captured images, a direction of patient support movement corresponds to a fixed vector direction. In response to patient support motion, a motion field calculation is performed between sequential captured images. Areas with a motion field parallel to the fixed vector direction (and hence substantially no motion perpendicular to the fixed vector direction) are identified as patient setup features. This information can be used in various ways as part of an automated workflow solution.

## Description

### FIELD OF THE INVENTION

The invention relates to monitoring a patient during a medical scan, in particular to distinguish between the patient setup and other nearby objects such as personnel and static parts of the scanner.

### BACKGROUND OF THE INVENTION

In many medical imaging procedures, it is important to monitor a patient during the imaging procedure, for instance, to guide and automate the exam preparation phase, to monitor movement of the patient before and during the procedure or to monitor the well-being of the patient. For example, monitoring images, such as video images provided by cameras, such as wide field of view cameras, are used for the monitoring.

Imaging may also be used to monitor the workflow of the scanning procedure.

Camera-based workflow solutions require algorithm solutions for efficient and accurate filtering of relevant signals from background signals. Typically, camera-based workflow solutions consist of complicated nested ensembles of AI neural network detection algorithms. Ideally, all neural network algorithms are trained adequately to have a stable detection performance.

Given the complexity of an examination workflow scene, separation of the patient setup from the rest of the scene is one of the most important yet challenging tasks.

A concrete application where an accurate separation is needed is the detection of collisions. In this application, parts of the patient preparation scene that may collide with the gantry bore as a result of patient support motion need to be detected before the patient is moved to the target scanning position. In such an application, it is highly important to limit the number of false positives while keeping a low number of false negatives, in order to prevent the generation of numerous unnecessary alerts. Therefore, only the parts that are subject to patient support motion are relevant in this task.

The separation of these parts based only on static camera data (RGB or depth) is not a trivial task.

There is therefore a need for an imaging system which is able to distinguish between the patient setup and other objects, but with low computational complexity.

### SUMMARY OF THE INVENTION

The invention is defined by the claims.

According to examples in accordance with an aspect of the invention, there is provided an imaging system for capturing images of a patient during a medical scan using a medical scanner having a scanning system and a patient support, the imaging system comprising:
a camera for mounting in a fixed position relative to the scanning system and directed towards the patient support for capturing images; and
a processor, wherein the processor is configured to:
   allocate a direction of patient support movement to a fixed vector direction within the images captured by the camera;
   in response to patient support motion, perform a calculation of motion fields between sequential captured images;
   identify image areas with a motion field parallel to the fixed vector direction and
   output the identified image areas as an identification of patient setup features.

This imaging system identifies patient setup features using a simple motion field analysis between sequential images. The analysis is for example triggered by a signal from a patient support sensor. It provides a strong separation of the patient setup from nearby personnel, objects and static parts of the scanner. In particular, the background is stable, the patient setup is moving in a predefined direction, and operators are moving randomly, hence with an incoherent motion field. Such a separation of scene items is non-trivial but opens significant opportunity for improving scene understanding especially for safety-related features of a workflow camera solution. It also provides a means to significantly boost detection robustness against background noise. In medical imaging applications, where controlled patient support movements are involved, the known patient support movement creates a precisely defined motion field along a given motion vector.

The term "patient setup features" is intended to mean all subjects and objects that move with the patient support.

In the patient support coordinate system, the patient setup is a very stable part of the scene.. Projecting the actual measured flow of the whole scene during patient support motion onto the patient support motion vector thus enables a clean delineation and segmentation of the patient setup including all attached parts moving along the table motion vector. This scalar signal can be used to filter the actual full scene motion field.

The filtered motion field can then additionally function as a pseudo stereo signal to derive 3D information, but completely restricted to the relevant patient setup volume only. In particular, the motion signal between two frames can be used to generate 3D information: for each pixel the disparity is equal to the flow vector. This disparity value is combined with the known table motion to derive depth information. Alternatively, the 3D information could be independently provided by the camera itself (if it is a 3D camera). In that case, the two depth values can be combined - to increase the robustness or the accuracy.

The derived 3D information is uniquely selective as described above and as such cannot be easily produced with a typical depth sensor alone, such as a time of flight camera or a stereo camera.

The camera is for example configured to be positioned such that a row direction of the captured images is aligned with the direction of patient support movement. This simplifies the calculation of motion fields.

The processor may instead be configured to transform the camera images such that a row direction of the transformed images is aligned with the direction of patient support movement. This requires additional image processing, but simplifies the system installation.

In one example, the processor comprises an input for receiving patient support motion information from the medical scanner and thereby detecting the patient support motion. In another example, the processor is further configured to analyze the camera images to derive patient support motion information and to thereby detect the patient support motion. In this way, the system can be a standalone system that does not need to take inputs from the medical scanner.

The processor may be further configured to generate an area mask of the patient setup. This mask can be used to distinguish between subjects and objects that will move inside the bore and those that will not, hence allowing system to focus on the relevant image parts. This can improve the detection rate, for example better distinction between patient and subject, or better distinction between accessories connected to the patient and those not connected to the patient.

The processor may be further configured to calculate a depth signal for the patient setup. The flow analysis enables a depth estimation, which thus enables different objects to be distinguished. The camera may comprise a 3D camera, and a depth signal obtained from the depth camera may be combined with the calculated depth signal. This can be used to improve accuracy and/or robustness.

The processor may be further configured to perform patient related calculations or patient setup related calculations.

For example, the processor may be further configured to perform proximity calculations between the patient set up features and the scanning system for collision avoidance. Bore collision avoidance can thus be implemented as a safety feature. If a collision is detected or predicted, the system may automatically slow down the patient support to enable the system operator to make adjustments. Thus, the motion of the patient support may be controlled in dependence on the proximity calculations.

The invention also provides a medical scanner comprising:
a scanning system;
a patient support extending through the scanning system;
a drive system for driving the patient support through the scanning system; and
the imaging system as define above.

The camera of the imaging system is for example mounted relative to the scanning system with an orientation such that a row direction of the captured images is aligned with the direction of patient support movement. This simplifies the flow analysis.

The invention also provides an imaging processing method for processing images of a patient during a medical scan using a medical scanner having a scanning system, a patient support and a camera mounted in a fixed position relative to the scanning system and directed towards the patient support for capturing images, wherein the method comprises:
allocating a direction of patient support movement to a fixed vector direction within the images captured by the camera;
in response to patient support motion, performing a calculation of motion fields between sequential captured images;
identifying image areas with a motion field parallel to the fixed vector direction; and
outputting the identified image areas as an identification of patient setup features.

This method detects areas that are static in the reference frame of the patient support, hence patient setup features, based on simple image analysis.

The method may comprise comprising transforming the camera images such that a row direction of the transformed images is aligned with the direction of patient support movement. This enables the camera to be mounted in any orientation relative to the patient support.

The method may comprise receiving patient support motion information from the medical scanner to detect the patient support motion or analyzing the camera images to derive patient support motion information and thereby detect the patient support motion.

The method may further comprise:
generating an area mask of the patient setup; and/or
calculating a depth signal for the patient setup; and/or
performing patient related calculations or patient setup related calculations.

The invention also provides a computer program comprising computer program code which is adapted, when the program is run on the processor of the imaging system defined above, to implement the method above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows schematically an example of a medical scanner;
Fig. 2 shows the result of motion analysis; and
Fig. 3 shows an image processing method.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an imaging system for capturing images of a patient during a medical scan using a medical scanner having a scanning system and a patient support. A camera is fixed relative to the scanning system and captures images of the patient support. Within the captured images, a direction of patient support movement corresponds to a fixed vector direction. In response to patient support motion, a calculation of motion fields is performed between sequential captured images. Areas with a motion field parallel to the fixed vector direction are identified as patient setup features. Such areas have substantially no motion perpendicular to the fixed vector direction. There may be no motion in the perpendicular direction or a motion amount below a threshold. This information can be used in various ways as part of an automated workflow solution.

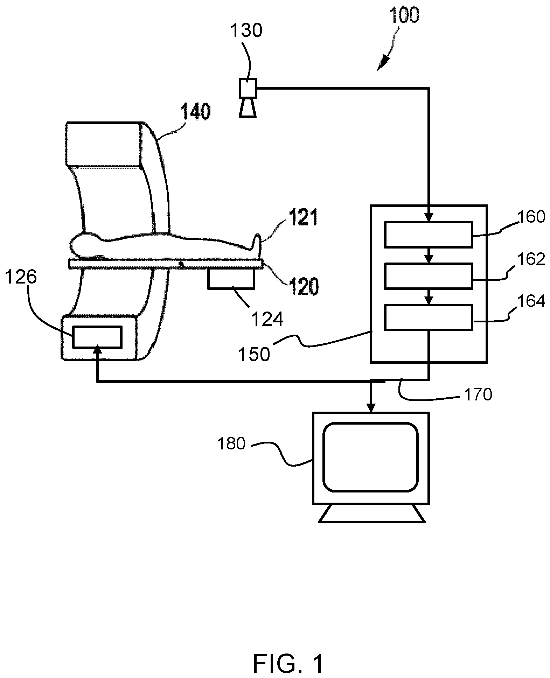

Fig. 1 shows schematically an example of a medical scanner 100 for acquiring a medical image of a subject and which additionally includes an imaging system for capturing optical images of the patient during the medical scan. The images are generated for the purposes of determining patient movement.

The medical scanner 100 comprises a scanning system such as a CT imaging system 140 that is adapted to acquire a medical image, i.e. a CT image in this example, of a patient 121 positioned on a patient support 120. The patient support 120 is adapted to move the patient 121 through the CT imaging system 140 during the CT imaging procedure. For this purpose, the medical scanner has a drive system 124 for driving the patient support through the scanning system.

The imaging system comprises an optical camera 130 that is adapted to acquire monitoring images of the patient 121 during the CT imaging procedure, and a processor 150. The operation of the system with a single camera will be explained, but there may be multiple cameras. The camera can be a color or monochrome camera. It can use visible light or infrared light. The camera may be a 2D imaging camera or it may be depth camera. As explained below, depth information may be obtained from a depth camera or by analysis of 2D images using the patient support motion as an additional input.

The camera 130 preferably has a wide field of view so that it captures a full view of the patient support 120, or at least the part of the patient support 120 over which regions of interest of the patient will be positioned. Furthermore, the movement of the patient support 120 means that an even wider field of view is needed in order for the desired parts of the patient support to remain in the field of view of the camera (which is mounted statically relative to the scanning system for example mounted to the ceiling) throughout the medical scan.

The camera for example comprises a fisheye lens with a field of view greater than 150 degrees, for example 160 degrees or more. Thus, the patient support (and the patient on the patient support) can be imaged either by a single camera as shown or by a small set of cameras.

In one example, the camera view has no perspective distortion with respect to the patient support so that the patient support movement is strictly along a prescribable vector, e.g. along the image x-axis.

However, in another example, any such distortion is corrected. For example, a wide angle lens results in image distortion, such that the shape of objects appears differently at different regions of the field of view of the camera. To address this issue, the processor 150 may perform image post-processing to correct those distortions within the captured images, i.e., resulting from the width of the field of view of the camera. This is shown as post-processing unit 160. For the purposes of the post-processing, the cameras is calibrated, such that geometric distortions are corrected by the post-processing, and the position and orientation of the camera with respect to the scanner coordinate system is known.

The camera 130 is mounted at a fixed location with respect to the static parts of the scanner. The resulting camera view, or else the camera image after distortion correction, is such that the patient support movement is strictly along a prescribable vector.

The camera position and orientation is calibrated with respect to the patient support of the scanner. This is known as computation of extrinsic parameters, or computation of the pose parameters of the camera. Based on the calibration, the exact pixel direction of a patient support motion is known.

It is preferable to transform the camera images so that the patient support motion is oriented strictly along the x-direction of the image, for example.

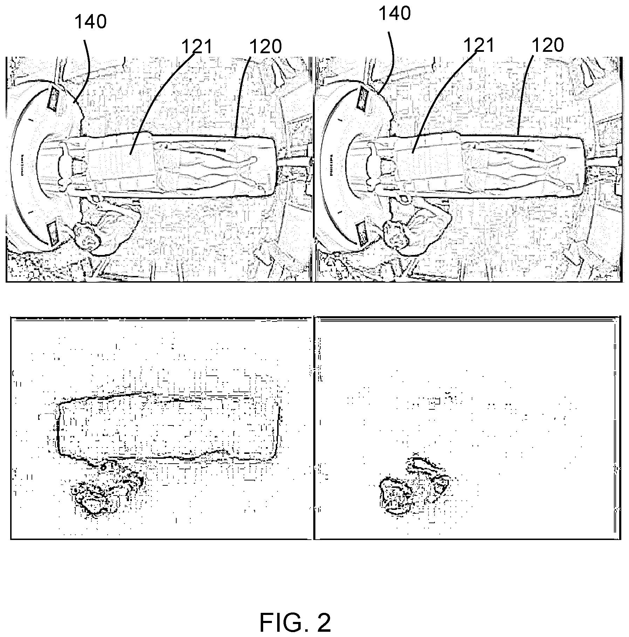

Fig. 2 shows a typical result of the calculation of motion fields. An optical flow calculation is one example of a suitable calculation of motion fields. For simplicity, the calculation of motion fields will be referred to as a "flow calculation" in the following text. The top images show the camera view of the patient 121 on the patient support 120.

The left image at the bottom shows the result of a flow calculation in a flow direction parallel to the fixed vector direction (i.e. in the patient support movement direction) and the right image at the bottom shows the result of a flow calculation in a flow direction perpendicular to the fixed vector direction.

As shown, the patient support has flow (i.e. motion) in the parallel direction but no flow (i.e. motion) in the perpendicular direction. Thus, the patient support has motion parallel to the vector direction. A system operator can be seen in both flow calculation images. The operator is active near the patient support, for example simultaneously pressing the patient support motion button to move the patient support in. Thus, there is clean x-direction flow (indicating x-direction motion) only for the patient setup area including all parts connected to the patient and patient support. In particular, there is a strong x-flow component in combination with a total (or near total) absence of y-flow component for the complete patient setup, whereas the operator area has mixed y- and x-flow, representing incoherent motion.

An absence of y-flow components (or more generally y-flow components below a threshold level) is extremely unlikely for the operator area while it is very likely for the patient setup, since this is the most stabilized part of the scene.

The flow calculation result has sub-pixel sensitivity. In addition to the clear separation of operator and patient setup, there is no spurious signal from any background objects such as monitor stands, gantry displays or the magnet itself. This shows the power of the signal for the intended purpose of separating patient setup from the background.

When a patient support motion event is reported or detected, the flow calculation takes place between consecutive frames. Image areas are then identified, and these represent patient setup features.

The camera should be capable to measure at sufficient frame rate to follow the patient support motion, e.g. ideally mm range inter-frame patient support motion. The system either has connectivity with the scanner system software and hardware control units to obtain information about the patient support motion or if it is a separated system with no connection, it has its own AI-based system to detect the patient support and motion events. Thus, the medical scanner either reports the patient support motion via separate system hardware components or the patient support motion is detected via a dedicated algorithm.

The detection and quantification of patient support motion events triggers the execution of the flow calculations immediately, so that short detection latencies can be achieved such as 50ms at 20Hz frame rate.

The identification of patient setup features may be provided for visually informing a user, or it may be in a machine-readable form for the purposes of controlling the medical scanner or other apparatus.

The information can for example be used to generate an area mask of the patient setup including all connected parts moving with the setup. The mask distinguishes between subjects and objects that will move inside the bore and those that will not, hence allowing system to focus on the relevant image parts.

A depth signal for the patient setup may also be generated, again including all connected parting moving with the setup. The signal may then be highly selective and can be used to compute or refine patient related calculations e.g., iso-center, pose, or patient setup related calculations, e.g. bore proximity calculations. Bore proximity calculations are for example of use for collision detection, to detect an unsafe patient setup condition.

The area mask is for example applied to select signals from other sensors for further analysis, such as to select those depth sensor signals which are of interest. - The patient set-up is observed by the camera, typically mounted above the patient support. In the case of a 3D camera, the video images (including color and depth) are analyzed to detect the patient and the patient accessories. This image analysis can then be used to check whether the set-up is complete, suitable, and safe.

In the case of a 2D camera, depth information can be obtained based on an analysis of the 2D images in combination with the movement information of the patient support. This approach for obtaining depth information is for example known from WO 2019/076734. It discloses a system and method to determine a height profile from images taken of a patient on a moving patient support.

All subjects and objects in the camera field of view are analyzed. Some objects will be located on the patient support and will move with the patient support inside the bore: typically, the patient, the coils, the positioning supports, and physiology sensing devices. Some objects will not move with the patient support: the operator, the hanging injection device, the physiology monitor, displays, etc.

Being able to separate the subjects and objects that will move inside the bore allows the image processing to focus on the relevant parts and ignore the others. Typically, this can improve the detection rate, for example to give a better distinction between the patient and other subject and a better distinction between accessories connected to the patient from those which are not.

For collision detection with the scanner bore, the system should be able to detect parts that will collide with the bore because of patient support displacement before the time of collision. Obviously, only the subject and objects moving with the couch are relevant for this analysis. By improving this distinction, the number of false positives in a collision detection system is reduced.

In the case of a detected or predicted collision, the patient support may be slowed down automatically, so that the operator can take action.

As mentioned above, the system preferably makes use of depth data, in particular to make collision detection robust. The depth data can come either from a depth sensor (e.g., time-of-flight sensor) or from the flow analysis of conventional (RGB) images, or from a combination of both.

It is noted that the collision detection use-case is extremely challenging for image guided therapy patient setup scenarios that typically have very crowded and busy workspaces with large number of peripheral equipment or equipment subparts and large number of nearby standing personnel. The proposed invention is extremely useful to ensure safe detector rotations avoiding collisions with the patient setup.

The invention may be applied to any medical scanner in which a patient support is moved during the imaging procedure, like a PET imaging device, an MR imaging device, a SPECT imaging device, as well as a CT scanner as described above. The medical scanner may comprise a C-arm or a closed bore. The camera or cameras may be located in the bore or at an inner surface of the C-arm, or outside the bore or C-arm envelope. However, in all cases, the camera is static relative to the main body of the scanning system and thus the patient support moves relative to the camera. The camera may be mounted to the room in which the scanner is housed or mount directly to the medical scanner.

Although in the above described embodiments the patient support is always a patient support on which a patient is lying during the acquisition of the medical image, the patient support can also be configured for a sitting or a standing patient.

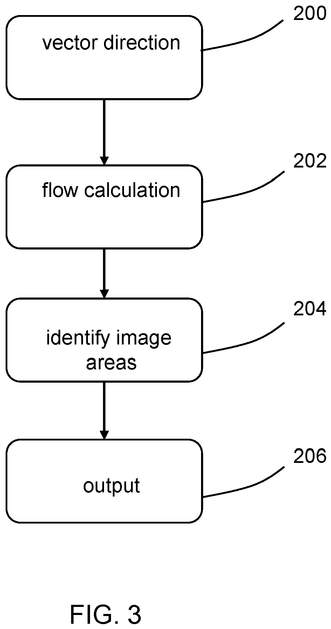

Fig. 3 shows an image processing method for processing images of a patient during a medical scan using a medical scanner having a scanning system and a camera.

In step 200, a direction of patient support movement is allocated to a fixed vector direction within the images captured by the camera.

In step 202, in response to patient support motion, a motion field calculation is carried out between sequential captured images.

In step 204, image areas are identified with motion parallel to the fixed vector direction.

In step 206, the identified image areas are output as an identification of patient setup features.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. An imaging system for capturing images of a patient during a medical scan using a medical scanner having a scanning system (140) and a patient support (120), the imaging system comprising:
a camera (130) for mounting in a fixed position relative to the scanning system and directed towards the patient support for capturing images; and
a processor (150), wherein the processor is configured to:
(200) allocate a direction of patient support movement to a fixed vector direction within the images captured by the camera;
(202) in response to patient support motion, perform a calculation of motion fields between sequential captured images;
(204) identify image areas with a motion field parallel to the fixed vector direction; and
(206) output the identified image areas as an identification of patient setup features.

2. The imaging system of claim 1, wherein:
the camera (130) is configured to be positioned such that a row direction of the captured images is aligned with the direction of patient support movement; or
the processor (150) is configured to transform the camera images such that a row direction of the transformed images is aligned with the direction of patient support movement.

3. The imaging system of any one of claims 1 to 2, wherein:
the processor (150) comprises an input for receiving patient support motion information from the medical scanner and thereby detect the patient support motion; or .
the processor (150) is further configured to analyze the camera images to derive patient support motion information and thereby detect the patient support motion.

4. The imaging system of any one of claims 1 to 3, wherein the processor (150) is further configured to generate an area mask of the patient setup based on the patient support motion.

5. The imaging system of any one of claims 1 to 4, wherein the processor (150) is further configured to calculate a depth signal for the patient setup.

6. The imaging system of claim 5, wherein the camera comprises a 3D camera, and wherein a depth signal obtained from the depth camera is combined with the calculated depth signal.

7. The imaging system of any one of claims 1 to 6, wherein the processor (150) is further configured to perform patient related calculations or patient setup related calculations.

8. The imaging system of any one of claims 1 to 7, wherein the processor (150) is further configured to perform proximity calculations between the patient set up features and the scanning system for collision prevention, and optionally to control the motion of the patient support in dependence on the proximity calculations.

9. A medical scanner comprising:
a scanning system (140);
a patient support (120) extending through the scanning system;
a drive system (124) for driving the patient support through the scanning system; and
the imaging system of any one of claims 1 to 8.

10. The medical scanner of claim 9, wherein the camera (130) of the imaging system is mounted relative to the scanning system with an orientation such that a row direction of the captured images is aligned with the direction of patient support movement.

11. An image processing method for processing images of a patient during a medical scan using a medical scanner having a scanning system, a patient support and a camera mounted in a fixed position relative to the scanning system and directed towards the patient support for capturing images, wherein the method comprises:
(200) allocating a direction of patient support movement to a fixed vector direction within the images captured by the camera;
(202) in response to patient support motion, performing a calculation of motion fields between sequential captured images;
(204) identifying image areas with a motion field parallel to the fixed vector direction; and
(206) outputting the identified image areas as an identification of patient setup features.

12. The method of claim 11, comprising transforming the camera images such that a row direction of the transformed images is aligned with the direction of patient support movement.

13. The method of claim 11 or 12, comprising receiving patient support motion information from the medical scanner to detect the patient support motion or analyzing the camera images to derive patient support motion information and thereby detect the patient support motion.

14. The method of any one of claims 11 to 13, further comprising:
generating an area mask of the patient setup; and/or
calculating a depth signal for the patient setup; and/or
performing patient related calculations or patient setup related calculations.

15. A computer program comprising computer program code which is adapted, when the program is run on the processor of the imaging system of any one of claims 1 to 10, to implement the method of any one of claims 11 to 14.
